# EUROPÄISCHE PATENTSCHRIFT

(11) **EP 2 161 607 B1**
(45) Veröffentlichungstag und Bekanntmachung des Hinweises auf die Patenterteilung: **10.07.2013**
(21) Anmeldenummer: 09175365.7
(22) Anmeldetag: 10.09.2006
(51) Int. Cl.: G02B 23/24, G02B 3/14, A61B 1/00

(54) **Dentalkamera**
Camera
Caméra

(30) Priorität: 27.10.2005 DE 102005051714
(43) Veröffentlichungstag der Anmeldung: 10.03.2010
(62) Teilanmeldung aus: 06018926.3
(73) Patentinhaber: LINOS Photonics GmbH & Co. KG, 37081 Göttingen (DE)
(72) Erfinder: Guse, Frank, 63454 Hanau (DE); Bürckner-Koydl, Dieter, 86415 Mering (DE); Partheymüller, Ulrich, 83607 Holzkirchen (DE); Knoch, Stefan, 81547 München (DE)
(74) Vertreter: Wagner, Carsten

(56) Entgegenhaltungen:
- WO-A2-02/096277
- DE-U1- 29 824 899
- FR-A1- 2 769 375
- US-A1- 2004 228 003
- Anonymous: "Philips' Fluid Lenses" Internet 3. März 2004 (2004-03-03), XP002565499 Gefunden im Internet: URL:http://www.dpreview.com/news/0403/0403 0302philipsfluidlens.asp> [gefunden am 2010-01-01]

## Beschreibung

Die Erfindung betrifft eine gattungsgemäße Dentalkamera gemäß dem Oberbegriff des Hauptanspruchs, nämlich eine solche mit einem Gehäuse, mit einem darin angeordneten, ein Objekt auf einen Bildabnehmer abbildenden Objektiv, mit einer dessen Fokussierung elektrisch ändernden Fokussiereinrichtung und mit einer Steuerung für diese.

Eine solche Dentalkamera ist bekannt (DE 101 25 772 A1). Bei dieser bekannten Dentalkamera wird ein von einer als an dem Gehäuse angebrachten Handhabe ausgebildeten Steuerung gesteuerter Elektromotor vorgesehen, dessen Achse mit einer Verstellspindel versehen ist. Diese ist in einem Stellglied geführt, an welchem der Bildabnehmer festgelegt ist und aufgrund der Drehung der Achse des Elektromotors verstellt werden kann, so dass durch die Fokussiereinrichtung die Scharfstellung der Abbildung des Objektivs durch die Relativlage von zumindest einer Linse zu den anderen Linsen des Objektivs oder dessen Relativlage zu dem Bildabnehmer geändert werden kann.

Von Nachteil bei dieser bekannten Dentalkamera ist die Tatsache der Notwendigkeit sowohl des gesonderten Stellgliedes als auch der Verstellspindel, neben dem Elektromotor selbst, welche Teile zusätzlichen Bauraum im Inneren des Gehäuses benötigen. Hierdurch wird verhindert, dass die Dentalkamera klein baut. Außerdem wird dadurch das Design und die Ergonomie solcher Dentalkameras negativ beeinflusst, wenngleich die Fokussierung damit in einfacher Weise zu bewerkstelligen ist.

Der Erfindung liegt die Aufgabe zugrunde, eine gattungsgemäße Dentalkamera so weiterzubilden, dass sie kleinbauend ausgestaltet ist.

Diese Aufgabe wird bei einer gattungsgemäßen Dentalkamera gemäß dem Oberbegriff des Hauptanspruchs erfindungsgemäß durch dessen kennzeichnende Merkmale also dadurch gelöst, dass die Fokussiereinrichtung ein mittels einer elektrischen Spannung in seinen Abbildungseigenschaften veränderbares elektrooptisches Bauelement, vorzugsweise eine mittels einer elektrischen Spannung in ihren Abbildungseigenschaften veränderbare variable Flüssiglinse aufweist.

Erfindungsgemäß wird in Abweichung vom Stand der Technik anstelle der hierbei verwendeten, elektromotorisch verstellbaren Stellglieder etc. also erfindungsgemäß eine Fokussiereinrichtung mit einem elektrooptischen Bauelement, vorzugsweise mit einer variablen Flüssiglinse eingesetzt, die elektronisch durch die Steuerung so gesteuert wird, dass ihre Abbildungseigenschaften in Form der Fokussierung auf verschiedene Objektabstände geändert werden, ohne dass Teile des Objektivs bewegt oder die Relativlage zu dem Bildabnehmer geändert werden müssen.

Mit der Erfindung wird also der Vorteil erzielt, dass in dem Bauraum der Dentalkamera weder ein Elektromotor noch eine Drehspindel noch ein zusätzliches Stellglied, sondern nur eine ohnehin notwendige Linse als elektrooptisches Bauelement in dem Objektiv als variable Flüssiglinse vorgesehen werden muss, insoweit also kein zusätzlicher Bauraum erforderlich ist. Infolgedessen ergibt sich insbesondere bei kleinen Kameras wie Dentalkameras der Vorteil einer elektronischen Fokussierung, ohne die Größe des Bauraums und die dadurch bedingte Ergonomie der Dentalkamera negativ zu beeinflussen. Außerdem erfolgt die Verstellung praktisch leistungslos, also ohne eine solche elektrische Verlustleistung, wie sie ein Elektromotor benötigt.

Es ist vorteilhaft, wenn die variable Flüssiglinse im Bereich unmittelbarer Nähe einer Aperturblende oder deren Bildes angeordnet ist, weil dann eine besonders kleine Bauform der Dentalkamera erzielt werden kann.

In zweckmäßiger Ausgestaltung der Erfindung kann in dem Objektiv eine verstellbare Blende vorgesehen sein, deren Durchmesser in einem vorgebbaren, bestimmten Verhältnis zur Fokuslage einstellbar ist. Durch die Fokussierung kann zugleich ein dementsprechendes Abblenden von einer kleinen Öffnung der Blende bei kleinem Aufnahmeabstand auf eine große Öffnung bei einem großen Aufnahmeabstand mit dem einstellbaren Verhältnis von Durchmesser zur Fokuslage erfolgen. Der Antrieb wird dabei elektrisch und automatisch entsprechend der Fokuslage betätigt.

Entweder ist diese verstellbare Blende einerseits mit dem Gehäuse und andererseits mit einem Antrieb - oder umgekehrt - jeweils fest verbunden und z.B. als Irisblende ausgebildet.

Besonders einfach wird die Einstellung der verstellbaren Blende, wenn diese als Flüssigkristallblende (LCD) ausgebildet ist und - ebenfalls praktisch leistungslos - über eine weitere elektrische Spannung eingestellt wird. Außerdem ergibt sich auch hier der Vorteil, dass keine Wärme durch elektrische Verlustleistung, wie bei einem Elektromotor erzeugt wird.

In bevorzugter Weiterbildung der Erfindung ist die Steuerung als Prozessor des Bildabnehmers zur Ermittlung der Bildschärfe ausgebildet, dessen Ausgang die elektrische Spannung für die variable Flüssiglinse führt und der Ausgang ist an die variable Flüssiglinse angeschlossen, weil dann manuell eine Einstellung des Fokus nicht erforderlich ist, vielmehr selbsttätig erfolgt. Dabei ist gar keine Handhabe zur Fokussierung mehr erforderlich, was die Möglichkeit der Reinigung der Kamera sowie Desinfektion bei der Ausbildung und Verwendung als Dentalkamera erheblich vereinfacht.

Weitere zweckmäßige Ausgestaltungen und Weiterbildungen der Erfindung sind in den weiteren Unteransprüchen gekennzeichnet.

Ein Ausführungsbeispiel der Erfindung wird nachfolgend unter Bezugnahme auf die Zeichnung näher erläutert, die die Dentalkamera in einem schematischen Halbschnitt zeigt.

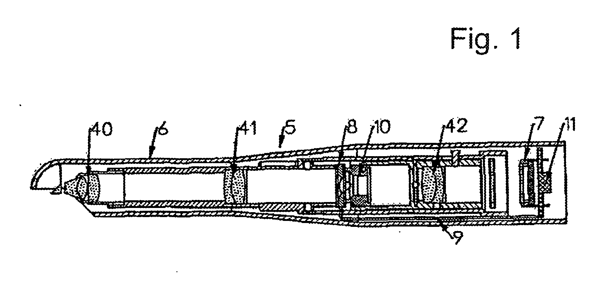

Die in Figur 1 insgesamt mit 5 bezeichnete Dentalkamera weist ein Gehäuse 6 mit einem darin angeordneten Objektiv 40,41,42 mit Linsen 40,41,42 auf, welches ein Objekt auf einen Bildabnehmer 7 abbildet. Hierbei weist das Objektiv 40,41,42 ein zwischen dem Objekt und dem Bildabnehmer 7 liegendes reelles Zwischenbild erzeugendes Teilobjektiv 40 auf und ist mit einer weiteren optischen Anordnung 41,42 versehen, welche dann das reelle Zwischenbild auf den Bildabnehmer 7 abbildet. Die weitere optische Anordnung 41,42 weist eine benachbart zu dem Teilobjektiv 40 angeordnete erste 41 und eine davon beabstandet angeordnete zweite Linsengruppe 42 auf.

In dem Gehäuse 6 ist eine als mittels einer elektrischen Spannung in ihren Abbildungseigenschaften veränderbare variable Flüssiglinse 8 - als Teil des Objektivs - ausgebildete, dessen Fokussierung elektrisch ändernde Fokussiereinrichtung vorgesehen, die von einer Steuerung gesteuert ist. Mit 9 ist die elektrische Zuführung zu der variablen Flüssiglinse 8 dargestellt. Die variable Flüssiglinse 8 ist hierbei zwischen der ersten 41 und der zweiten Linsengruppe 42 angeordnet.

Ferner weist das Gehäuse 6 der Dentalkamera 5 in dem Objektiv eine verstellbare Blende 10 auf, deren Durchmesser in einem bestimmten Verhältnis zur Fokuslage einstellbar und als Flüssigkristallblende (LCD) ausgebildet ist, die über eine weitere elektrische Spannung gesteuert ist. Dieser Anschluss ist aus Gründen der Übersichtlichkeit in der Zeichnung nicht dargestellt.

Der Bildabnehmer 7 ist ferner mit der als Prozessor 11 zur Ermittlung der Bildschärfe ausgebildeten Steuerung versehen, dessen Ausgang die elektrische Spannung für die variable Flüssiglinse 8 führt und an deren Eingang angeschlossen ist. Diese Ausbildung der Kamera eignet sich insbesondere für den Einsatz als Dentalkamera.

## Patentansprüche

1. Dentalkamera (5) mit einem Gehäuse (6), mit einem darin angeordneten, ein Objekt auf einen Bildabnehmer (7) abbildenden Objektiv (40,41,42), mit einer dessen Fokussierung elektrisch ändernden Fokussiereinrichtung und mit einer Steuerung für diese, wobei das Objektiv (40, 41,42) ein zwischen dem Objekt und dem Bildabnehmer (7) liegendes reelles Zwischenbild erzeugendes Teilobjektiv (40) aufweist, **dadurch gekennzeichnet, dass** die Fokussiereinrichtung ein mittels einer elektrischen Spannung in seinen Abbildungseigenschaften veränderbares elektrooptisches Bauelement (8) aufweist, welches als variable Flüssiglinse ausgebildet ist, wobei die variable Flüssiglinse (8) im Bereich unmittelbarer Nähe einer Aperturblende (10) oder im Bereich unmittelbarer Nähe eines Bildes einer Aperturblende (10) angeordnet ist.

2. Dentalkamera (5) nach Anspruch 1, **dadurch gekennzeichnet, dass** die variable Flüssiglinse zwischen dem reellen Zwischenbild und dem Bildabnehmer (7) angeordnet ist.

3. Dentalkamera (5) nach Anspruch 1 oder 2, **dadurch gekennzeichnet, dass** das Objektiv (40,41,42) eine weitere optische Anordnung (41,42) aufweist, welche das reelle Zwischenbild auf den Bildabnehmer (7) abbildet.

4. Dentalkamera (5) nach Anspruch 3, **dadurch gekennzeichnet, dass** die weitere optische Anordnung (41,42) eine benachbart zu dem Teilobjektiv (40) angeordnete erste (41) und eine davon beabstandet angeordnete zweite Linsengruppe (42) aufweist.

5. Dentalkamera (5) nach Anspruch 4, **dadurch gekennzeichnet, dass** die variable Flüssiglinse (8) zwischen diesen Linsengruppen angeordnet ist.

6. Dentalkamera (5) nach einem der vorhergehenden Ansprüche, **dadurch gekennzeichnet, dass** in dem Objektiv (40,41,42) eine verstellbare Blende (10) vorgesehen ist.

7. Dentalkamera (5) nach Anspruch 6, **dadurch gekennzeichnet, dass** der Durchmesser der verstellbaren Blende (10) in einem bestimmten Verhältnis zur Fokuslage einstellbar ist.

8. Dentalkamera (5) nach einem der vorhergehenden Ansprüche, **dadurch gekennzeichnet, dass** die Steuerung als Prozessor (11) des Bildabnehmers (7) zur Ermittlung der Bildschärfe ausgebildet ist.

9. Dentalkamera (5) nach Anspruch 8, **dadurch gekennzeichnet, dass** der Ausgang des Prozessors (11) die elektrische Spannung für die variable Flüssiglinse führt und der Ausgang an die variable Flüssiglinse angeschlossen ist.

## Claims

1. Dental camera (5) comprising a housing (6) with a lens (40, 41, 42) arranged therein for displaying an object on an image receiver (7), with a focussing device changing the focus electrically and with a control for the latter, wherein the lens (40, 41, 42) comprises a part lens (40) producing a real intermediate image between the lens and the image receiver (7), **characterised in that** the focussing device comprises an electro-optical component (8), the imaging properties of which can be changed by means of electric voltage, which component is configured as a variable liquid lens, wherein the variable liquid lens (8) is arranged in the area immediately adjacent to an aperture diaphragm (10) or in the area immediately adjacent to an image of an aperture diaphragm (10).

2. Dental camera (5) according to claim 1, **characterised in that** the variable liquid lens is arranged between the real intermediate image and the image receiver (7).

3. Dental camera (5) according to claim 1 or 2, **characterised in that** the lens (40, 41, 42) comprises an additional optical arrangement (41, 42) which displays the real intermediate image on the image receiver (7).

4. Dental camera (5) according to claim 3, **characterised in that** the additional optical arrangement (41, 42) comprises a first lens group (41) arranged adjacent to the part lens (40) and a second lens group (42) arranged spaced apart therefrom.

5. Dental camera (5) according to claim 4, **characterised in that** the variable liquid lens (8) is arranged between said lens groups.

6. Dental camera (5) according to one of the preceding claims, **characterised in that** an adjustable diaphragm (10) is provided in the lens (40, 41, 42).

7. Dental camera (5) according to claim 6, **characterised in that** the diameter of the adjustable diaphragm (10) can be adjusted in a specific ratio to the focus position.

8. Dental camera (5) according to one of the preceding claims, **characterised in that** the control is configured as a processor (11) of the image receiver (7) for determining the image sharpness.

9. Dental camera (5) according to claim 8, **characterised in that** the output of the processor (11) carries the electric voltage for the variable liquid lens and the output is connected to the variable liquid lens.

## Revendications

1. Caméra dentaire (5) comportant un boîtier (6), comportant un objectif (40, 41, 42) agencé dans ce dernier et reproduisant un objet sur un récepteur d'image (7), comportant un dispositif de focalisation modifiant électriquement la focalisation dudit récepteur d'image, et comportant une commande pour ledit dispositif de focalisation, ledit objectif (40, 41, 42) comportant un objectif partiel (40) générant une image intermédiaire réelle située entre l'objet et le récepteur d'image (7), **caractérisée en ce que** le dispositif de focalisation comporte un élément électro-optique (8), dont les propriétés de reproduction peuvent être modifiées au moyen d'une tension électrique et qui est conçu sous forme de lentille liquide variable, ladite lentille liquide (8) variable étant agencée dans la zone à proximité immédiate d'un diaphragme d'ouverture (10) ou dans la zone à proximité immédiate d'une image d'un diaphragme d'ouverture (10).

2. Caméra dentaire (5) selon la revendication 1, **caractérisée en ce que** la lentille liquide variable est agencée entre l'image intermédiaire réelle et le récepteur d'image (7).

3. Caméra dentaire (5) selon la revendication 1 ou 2, **caractérisée en ce que** l'objectif (40, 41, 42) comporte un agencement optique (41, 42) supplémentaire, qui reproduit l'image intermédiaire réelle sur le récepteur d'image (7).

4. Caméra dentaire (5) selon la revendication 3, **caractérisée en ce que** l'agencement optique (41, 42) supplémentaire comporte un premier groupe de lentilles (41), agencé à proximité de l'objectif partiel (40) et un deuxième groupe de lentilles (42) écarté dudit premier groupe.

5. Caméra dentaire (5) selon la revendication 4, **caractérisée en ce que** la lentille liquide (8) variable est agencée entre lesdits groupes de lentilles.

6. Caméra dentaire (5) selon l'une quelconque des revendications précédentes, **caractérisée en ce qu'**un diaphragme (10) réglable est prévu dans l'objectif (40, 41, 42).

7. Caméra dentaire (5) selon la revendication 6, **caractérisée en ce que** le diamètre du diaphragme (10) réglable peut être réglé dans un rapport déterminé par rapport à la position du foyer.

8. Caméra dentaire (5) selon l'une quelconque des revendications précédentes, **caractérisée en ce que** la commande est réalisée sous forme de processeur (11) du récepteur d'image (7) pour déterminer la netteté de l'image.

9. Caméra dentaire (5) selon la revendication 8, **caractérisée en ce que** la sortie du processeur (11) conduit la tension électrique pour la lentille liquide variable et ladite sortie est raccordée à ladite lentille liquide variable.
